Europäisches Patentamt

European Patent Office

Office européen des brevets

(11) Publication number: **0 448 795 A2**

# EUROPEAN PATENT APPLICATION

(21) Application number: 90123434.4

(22) Date of filing: 06.12.90

(51) Int. Cl.5: **B01L 3/14**

(30) Priority: 29.03.90 US 501214

(43) Date of publication of application:
02.10.91 Bulletin 91/40

(84) Designated Contracting States:
AT BE CH DE DK ES FR GB GR IT LI LU NL SE

(71) Applicant: **Becton Dickinson and Company**
**One Becton Drive**
**Franklin Lakes New Jersey 07417-1880(US)**

(72) Inventor: **Dufresne, Christopher M.**
**27 Millington Avenue**
**Hewitt, New Jersey(US)**

(74) Representative: **Selting, Günther, Dipl.-Ing. et al**
**Patentanwälte von Kreisler, Selting, Werner**
**Deichmannhaus am Hauptbahnhof**
**W-5000 Köln 1(DE)**

(54) Compartmental body fluid collection tube.

(57) A compartmented body fluid specimen collection tube is provided which allows the user to select a size compartment depending upon the quantity of specimen required. Thus, the tube of the invention may be of the standard size which is much easier for the user to handle than very small tubes when taking a blood sample, but a selected size compartment is available. The manufacturer of the tube itself may use a single mold for making tubes accommodating differing required specimen quantities, and, thereafter the vendor may select either size compartment for imparting a vacuum and the other processing required into a final product. The invention allows the use of a single standard size laboratory equipment array for processing collected specimens of varying quantities, as well as a single standard stopper. Finally, reduced vacuum application or "partial draw" tubes are avoided, thus eliminating the problem of unpredictable specimen draw response because of varying atmospheric pressures at different locations of use.

FIG-2

## BACKGROUND AND STATEMENT OF THE INVENTION

This invention relates to body fluid collection tubes for collecting a body fluid specimen from a patient for subsequent examination in a laboratory. More particularly, this invention relates to a blood collection tube for taking such specimens in which the tube has imparted thereto an internal vacuum with the vacuum being utilized for drawing the blood specimen from the vein of a patient.

Attempts have been made to overcome the problem of proliferation of a number of tubes for taking a plurality of specimens from a single venous entry into a patient. For example, Japanese Patent Application No. 62-51238, filed March 6, 1987 teaches a device in which a tube is divided axially into two chambers for taking two blood specimens simultaneously. However, there is no recognition for arranging different size compartments for taking different separate specimens of different size to accommodate subsequent laboratory testing of those specimens.

Other prior art arrangements, such as that taught in U.S. Patent 3,894,951, for example, provide for compartmentalized blood collection tubes in which the compartments have fluid connections for subsequent separation of a single blood sample into separate components.

In taking such specimens, differing quantities or volumes of specimens are required for certain applications. For this reason, larger and smaller tubes are manufactured for this purpose. As an alternative to making larger and smaller tubes, so-called "partial draw" tubes are provided in which the vacuum imparted to the tube is varied in order to accommodate differing quantities of specimen as required. All of these variations increase the cost of collecting blood specimens, and when it is understood that literally billions of such tubes are used and discarded after a single use each year the costs become substantial for medical applications.

This invention overcomes this problem by using a conventionally sized tube of, for example, 6cc in volume and incorporates an integral divider substantially centrally of the length of the tube to provide two compartments of differing size. Such compartments may be, for example, 2cc and 3cc. Thus, the manufacturer, rather than having to use two separate molds and two separate production lines, may manufacture a single tube having a selection of a 2cc and a 3cc compartment or chamber. Thereafter, the user or vendor may utilize or select one of the sizes of compartment and impart a vacuum thereto and place a stopper in the open end of the compartment size selected. The remaining compartment or chamber not being utilized for this particular series of tubes has a plug for closing the open end of the non-utilized chamber or compartment.

Conventional blood collection tubes have only one chamber, and volume in those conventional tubes is varied by using either larger or smaller diameter tubes, taller or shorter length tubes or varying the degree of vacuum imparted. As will be understood by practitioners-in-the-art, larger and smaller diameter tubes require specific dimensioned stoppers for accommodating those various sizes. Moreover, taller and shorter tubes require different packaging to accommodate tube length. If an accommodation is made to utilizing only a single size tube and imparting a partial vacuum for subsequent use in which only a small quantity of specimen is required in relatively large tubes, there is inconsistent draw over different areas of use because of differing atmospheric pressure gradients.

Finally, the users of tubes of different length and diameter must have varying size equipment, such as centrifuges, tube racks and like supporting arrangements to accommodate the varying sizes of tubes being utilized for collecting differing quantities of body fluid specimens for different applications.

With this invention, by contrast, a single size tube, as noted above is utilized. For this reason, the user, in the sometimes tense environment of taking a blood specimen from an individual, has a relatively large tube for handling during the collection activity Nevertheless the tube has a smaller compartment; for accommodating the actual size specimen required. Moreover, because only a single size tube relative to diameter is being utilized with the invention here, a single stopper size is required rather than a plurality of stopper sizes of differing diameters to accommodate differing diametered tubes. This reduces manufacturing requirements as well as storage requirements for such a variety of stopper sizes.

Moreover, because of the large size tubes having small compartmentalized areas, no "partial draw" vacuum applications are required. This eliminates the problem of inconsistency depending upon the atmospheric pressure gradients over many areas of use of such tubes. Finally, because of the uniformity of size of the tube, the hospital or clinical laboratory only needs to have one size equipment necessary for handling the tubes of a standard size.

A further feature of the invention may be the use of both compartments of a single tube by the phlebotomist. That is, the vendor or manufacturer of the tubes may, for example, impart vacuum to both a 3cc and a 2cc compartment or chamber in a single tube and place stoppers for sealing and maintaining the vacuum. Thereafter, the phlebotomist, in requiring two separate blood sample volumes for different tests subsequently can first draw blood into one chamber followed by inversion of the tube and collection of blood into the second chamber. Two blood samples would then be contained in a single tube for subsequent examination in the clinical laboratory, and with only one venous entry.

Other objects and advantages of this invention will be apparent from the following description, the accompanying drawings and the appended claims.

DESCRIPTION OF THE DRAWINGS

Fig. 1 is longitudinal sectional view of the compartmentalized blood collection tube of the invention;

Fig. 2 is a longitudinal sectional view of one embodiment of the invention wherein a vacuum has been imparted to the smaller chamber or compartment of the tube of the invention and showing the sealing stopper arrangement for such a tube, together with a plug for the unused chamber;

Fig. 3 is a longitudinal sectional view of the tube of the invention reversed in its application from that shown in Fig. 2;

Fig. 4 is an enlarged partial cross-sectional view of one open end of the tube of the invention showing the mating configuration of a sealing plug applied to the open end for closing off the non-utilized chamber or compartment of the tube of the invention;

Fig. 5 is a longitudinal sectional view of a blood sample tube holder for taking a blood sample from a vein of a patient with the tube of the invention inserted therein; and

Fig. 6 is a longitudinal sectional view of a tube similar to that shown in Fig. 1, but both chambers being closed with a stopper closure for use in evacuating two chambers for collecting two specimens sequentially.

DETAILED DESCRIPTION OF THE INVENTION

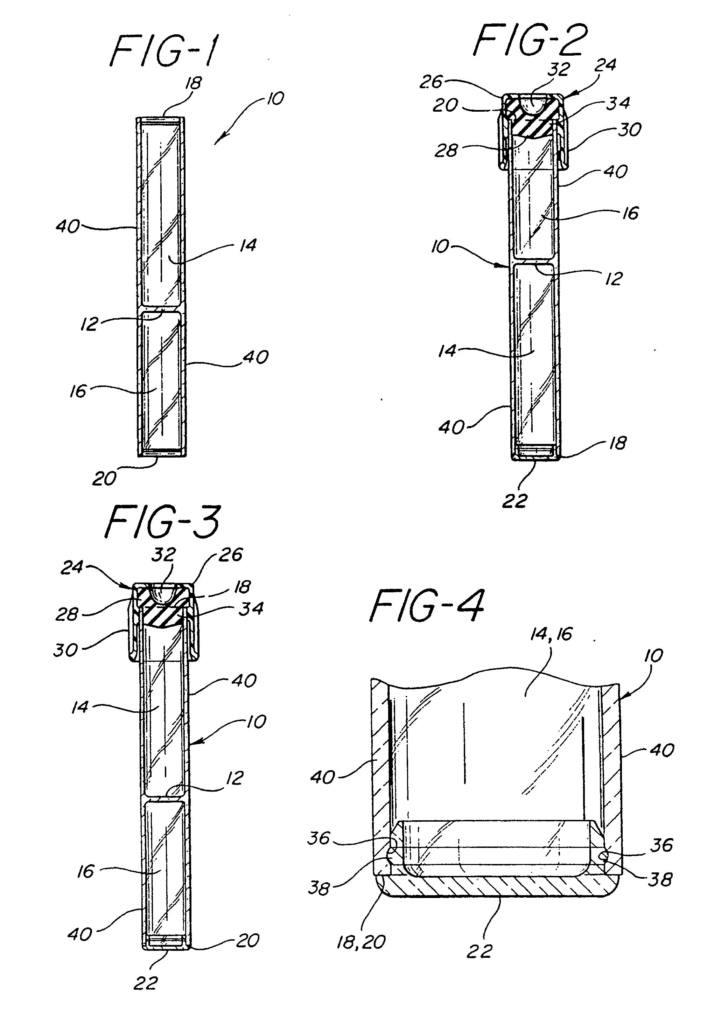

Referring to the drawings in which like reference characters refer to like parts throughout the several views thereof, Fig. 1 illustrates the invention in the form of a conventionally sized blood collection tube compartmentalized in its formation to provide two chambers or compartments of differing size to accommodate differing blood sample requirements.

In Fig. 1, the device 10 is an elongated tube with walls 40 and having open ends 18, 20 at each end thereof. As can be seen in Fig. 1, tube 10 is divided by a wall 12 into compartments or chambers 14 and 16. Also, as will be seen in Fig. 1, chamber 14 is larger than chamber 16. For example, in a 6cc tube, chamber 14 may be, for example, 3cc in volume while chamber 16 may be 2cc in volume.

It will be understood that the volume amounts may be varied as use requirements dictate. Tube 10 may be comprised of a thermoplastic resin material, for example, formed in a mold to provide wall 12 integrally joined to wall 40 of tube 10.

Referring now to Fig. 2, tube 10 is shown in a prepared form for use by a vendor. In this case, the smaller dimensioned chamber 16 has had, for example, a vacuum applied thereto with a subsequent insertion of a stopper assembly 24 for maintaining the vacuum in chamber 16. The assembly 24 includes a rubber stopper for closing the open end, 20 of the tube, assembly 10. That is, the outer surface of lower portion of stopper 28 seals against the internal surface of walls 40 of the upper end of tube 10. The assembly 24 includes a plastic cap 26 connected to and surrounding elastomer stopper 28. This serves to accommodate removal of stopper 28 in a simple fashion, when required, to obtain access to the collected sample in chamber 16.

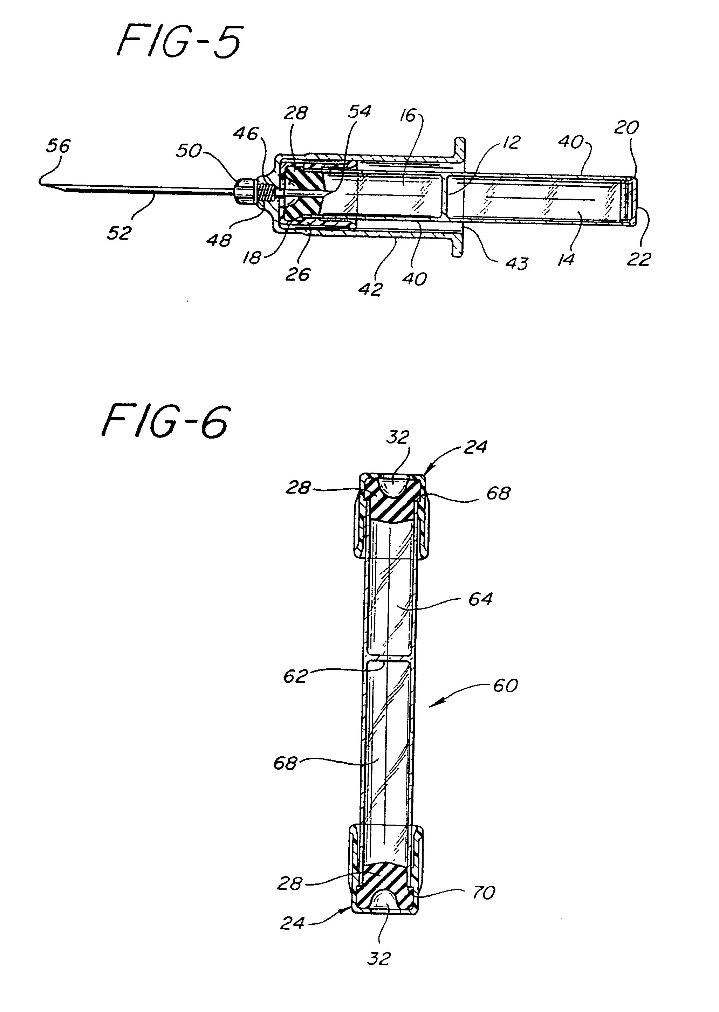

Stopper 28 includes a well 32 formed in the top surface thereof which has the purpose of reducing the vertical extent of stopper 28 in the center portion thereof to provide a diaphragm 34 of vertically smaller dimension through stopper 28 for receiving a needle such as needle 52 and the point 54 thereof, as shown in Fig. 5. The plastic portion 26 of stopper 24 assembly includes outer ridges 34 to provide an easier grip of the assembly 24 for removal thereof from the evacuated chamber 16, when required.

Referring to Fig. 4, the open end 18 of chamber 14, which in this case is not being utilized for fluid collection includes a plug 22 for stabilizing the open end 18 of the tube assembly 10 for subsequent use of the tube or for storing the tube prior to use.

In Fig. 4, an enlarged view of the open end 18 or 20 is shown to accommodate the snap-fit arrangement of plug 22 in the open end 18 or 20 of chamber 14 or 16, as the case may be, when they are not being utilized in a particular tube 10 assembly. That is, the lower end of wall 40 may include an annular indentation 36 for receiving in snap-fit engagement an annular abutment, 38 on plug 22.

Referring now to Fig. 3, a tube 10 is shown reversed from the Fig. 2 showing for accommodating use of the 3cc and/or larger compartment 14 as desired by the user. In this case, stopper assembly 24 closes the open end 18 rather than 20, while the open end 20 is closed with a plug 22. It will be understood by practitioners-in-the-art that both compartments or chambers 14, 16 may be utilized in a single tube 10 by imparting a vacuum to both chambers and using a stopper closure assembly 24 for closing both open ends 18 and 20. In such cases, a plug 22 is not utilized.

Such an arrangement is shown in Fig. 6 wherein a tube 60 similar to tube 10 is shown with chambers 64, 66 divided by wall 62. Tube 60 includes two open ends 68 and 70. In this case, a stopper assembly 24 is applied to both open ends after application of vacuum to chambers 64, 68, respectively. As discussed above, in such an application, the user collects two different volumes of specimen from a patient as required for subsequent testing purposes.

In use, the assembly 10 of the invention is inserted into a blood collection needle holder such as 42 shown in Fig. 5. Holders 42, for example, include at the closed end thereof a hub 46 with internal screw threads 48 for accommodating the screw threads of a needle holder hub 50. Thus, a plurality of needle holder hubs 50 may be, sequentially, screwed into hub 46 of needle holder 42 for repeated use. The needle 52 includes a sharpened end 56 which is inserted into the vein of a patient for collecting a blood Specimen.

Subsequent to insertion of the needle 56 into the vein of a patient, the laboratory technician, doctor or nurse inserts the tube assembly 10 into the blood collection holder 42 at the open end 43 thereof. In doing so, the technician holds onto the holder 42 utilizing the flange 44. Subsequently, the tube 10 is moved forward toward the closed end of the holder 42 until such time as the negative pressure needle point 54 passes through stopper 28. When this happens, access to the vacuum in chamber 16 takes place and the vacuum draws blood from the patient's vein through the needle 52 into chamber 16.

As will be understood by practitioners-in-the-art, the smaller chamber 16 may be, for example, of a volume of 2ccs which is all that is required for certain subsequent examinations of the specimen in the lab. A tube such as tube 60 shown in Fig. 6 may be used in holder 42 instead of tube 10. With such an arrangement, the double-ended needle assembly would include a sealing valve sleeve well known in the art so that the tube 60 may be pulled off needle point 54, and reversed for inserting the needle point 54 into the subsequent stopper 28 without blood loss, for collecting a second blood sample in chamber 66, for example.

Alternatively, the technician may use a standard size blood collection container and collect a subsequent larger blood sample, as required from the single insertion of point 56 of needle 52 in the patient's vein all as will be understood by the practitioner-in-the-art.

The blood collection tube of the invention may be comprised, preferably, from a clear molded thermoplastic material such as polyethylene, for example, or glass. Other materials which may be used, as will be appreciated by practitioners-in-the-art include various other thermoplastics such as polypropylene, polyethylene terephthalate, and polyvinyl chloride.

The evacuated chambers utilized for collection in the device of the invention here may have incorporated therein materials for reaction with the specimen being collected for providing a proper response to the collected specimen for subsequent examination in the clinical laboratory. The stopper may be comprised of an elastomer appropriate for providing the sealing required to maintain the imparted vacuum in the chamber selected for subsequent use by the laboratory technician. The plastic cover portion 26 of stopper assembly 24 may be comprised of polyethylene or other thermoplastic which may be molded into the proper shape for the assembly of the invention.

Whereas, as discussed above, specific embodiments of associated stoppers and caps have been shown, it should be understood that it is within the purview of this invention to provide other forms of closures for blood collection tubes of the kind discussed herein.

It will be understood that the invention here is most appropriate for medical applications because of the substantial reduced cost in accommodating a variety of different requirements for a body fluid specimen collection, and for reducing the number of molds required for providing an assembly which is nevertheless, much easier to handle than the miniaturized collection tubes presently being utilized for small volume specimen collection. Moreover, the arrangement herein eliminates the need for the application of "partial draw" tubes and the inconsistency apparent with the use of such partial draw tubes.

While the forms of apparatus herein described constitute preferred embodiments of the invention, it is to be understood that the invention is not limited to these precise forms of apparatus and that changes may be made therein without departing from the scope of the invention which is defined in the appended claims.

## Claims

1.  A blood collection tube; comprising

    (a) a tube body;

    (b) said tube body comprised of walls formed as a cylinder to define a tube body chamber;

    (c) said tube body chamber having a first open end at one end of said tube body chamber and a second open end at the end of said tube body chamber opposite said first open end;

(d) a divider wall positioned between said first and second open ends;

(e) said divider wall dividing said tube body chamber into a first and a second compartment; and

(f) said divider wall being formed integrally as one piece with said cylindrical tube body walls.

2. The blood collection tube of Claim 1, further comprising

(a) said first and second compartments being of different volume capacity.

3. The blood collection tube of Claim 1 further comprising

(a) a stopper closing one of said first and second open ends; and

(b) a plug closing the other of said open ends not closed by said stopper.

4. The blood collection tube of Claim 3, further comprising

(a) said compartment with the open end closed by said stopper being evacuated.

5. The blood collection tube of Claim 3, further comprising said stopper being comprised of

(a) a first elastomeric portion inserted into said open end in sealing engagement therewith;

(b) a plastic portion fitted over and surrounding said elastomeric portion;

(c) said plastic portion encompassing said tubular walls adjacent said open end into which said stopper is inserted;

(d) the top of said plastic portion having an opening adjacent the axis thereof for providing needle access to said elastomeric portion; and

(e) cooperating abutment means on said elastomeric portion and said plastic portion for holding said elastomeric portion and said plastic portion together.

6. The blood collection tube of Claim 3, further comprising

(a) said tube walls and said divider wall are comprised of a material selected from the group consisting of polyethylene, polypropylene, polyethylene terephthalate, polyvinyl chloride and glass.

7. A blood collection tube; comprising

(a) a tube body;

(b) said tube body comprised of walls formed as a cylinder to define a tube body chamber;

(c) said tube body chamber having a first open end at one end of said tube body chamber and a second open end at the end of said tube body chamber opposite said first open end;

(d) a divider wall positioned between said first and second open ends;

(e) said divider wall dividing said tube body chamber into a first and a second compartment;

(f) said divider wall being formed integrally as one piece with said cylindrical tube body walls;

(g) said first and second compartments being of different volume capacity;

(h) a first stopper closing said first open end; and

(i) a second stopper closing said second open end.

8. The blood collection tube of Claim 7, further comprising

(a) both said first and said second compartments being evacuated.

9. The blood collection tube of Claim 7, further comprising each of said first and second stoppers comprised of

(a) a first elastomeric portion inserted into said open end in sealing engagement therewith;

(b) a plastic portion fitted over and surrounding said elastomeric portion;

(c) said plastic portion encompassing said tubular walls adjacent said open end into which said stopper is inserted;

(d) the top of said plastic portion having an opening adjacent the axis thereof for providing needle access to said elastomeric portion; and

(e) cooperating abutment means on said elastomeric portion and said plastic portion for holding said elastomeric portion and said plastic portion together.

10. The blood collection tube of Claim 7, further comprising

(a) said tube walls and said divider wall are comprised of a material selected from the group consisting of polyethylene, polypropylene, polyethylene terephthalate, polyvinyl chloride and glass.

FIG-1

FIG-2

FIG-3

FIG-4

## FIG-5

## FIG-6